# EUROPEAN PATENT APPLICATION

(11) **EP 4 454 587 A1**
(43) Date of publication of application: **30.10.2024**
(21) Application number: 23382390.5
(22) Date of filing: 26.04.2023
(51) Int. Cl.: A61B 34/10

(54) **METHOD FOR MONITORING THE SPATIAL DEVIATION WITH RESPECT TO A PLANNED SURGICAL PROCEDURE**

(71) Applicant: Fundación para la Investigación Biomédica del Hospital Universitario Ramón y Cajal, 28034 Madrid (ES); Software Nemotec S.L., 28919 Leganés (Madrid) (ES)
(72) Inventor: ACERO SANZ, Julio Jesús, 28034 Madrid (ES); DEL MORAL SANCHEZ, Miguel, 28919 Madrid (ES)
(74) Representative: Hoffmann Eitle

(57) **Abstract**

The present invention relates to a computer-implemented method for monitoring the spatial deviation with respect to a planned surgical procedure, wherein the method is implemented through a system comprising at least a processor, the system characterised by the processor having access to a planned surgical procedure comprising one or more three-dimensional (3D) images corresponding to one or more predefined states of the planned surgical procedure in a body portion. The method comprises the following steps: a)receiving one or more image of the body portion obtained by an intraoperative imaging device wherein the one or more image is associated to a certain predefined state of the planned surgical procedure; b)superimposing the one or more received images of step a) with the corresponding section of the one or more 3D image of the planned surgical procedure associated to the same predefined state; and c) determining the spatial deviation of the surgical procedure results with respect to the planned surgical procedure from the superimposition of the images of step b). The present invention also refers to a computer program and a device thereof.

## Description

### Technical field of the invention

The present invention belongs to the field of medicine. Particularly, the present invention relates to the field of surgical planning, more particularly to a method for monitoring the spatial deviation with respect to a planned surgical procedure, to a computer program and to a system thereof.

### Background of the invention

Several surgical fields involve the correction of deformities acquired due to an alteration of the growth of tissues, particularly hard tissues such as bones. These produce not only an alteration in facial aesthetics, but also a functional problem and be associated to other physical and psychological problems. One of these fields is orthognathic surgery, and from herein after reference will be made to this field, although the same reasoning can be applied to other surgical fields such as congenital or post-trauma deformities or deformities after oncologic excision affecting any region of the skeleton.

Orthognathic surgery is one that allows the correction of orofacial deformities. These deformities are due to acquired alterations in the growth of the jaws and have a great prevalence producing not only an alteration in facial aesthetics, but also a functional problem usually with dental malocclusion and alterations in chewing, digestion of food and problems of the temporomandibular joint (pain, limitation in oral opening etc.). Occasionally, they can be associated with respiratory disorders such as OSAS (obstructive sleep apnea syndrome). Finally, self-perception disorders due to facial disharmony are also important. The incidence in Spain of these deformities is estimated at around 2-3% of the total population, assuming its correction one of the most frequent surgical procedures in the specialty of Oral and Maxillofacial Surgery, adding a total of 92 surgical procedures at the Ramón y Cajal Hospital in 2019.

There are three crucial aspects in the management of these patients that have changed substantially with technological advances: 1) planning surgery, 2) transfer of the planned movements to the surgical field, and 3) verification of the performed movements.

Currently, the gold standard in surgical planning is the three-dimensional study of the skeleton and facial soft tissues based on the image obtained by computed tomography (CT) and 3D virtual design on the surgical computer using osteotomies of the jaws. Planned movements are currently transferred by means of occlusal splints and sometimes cutting guides.

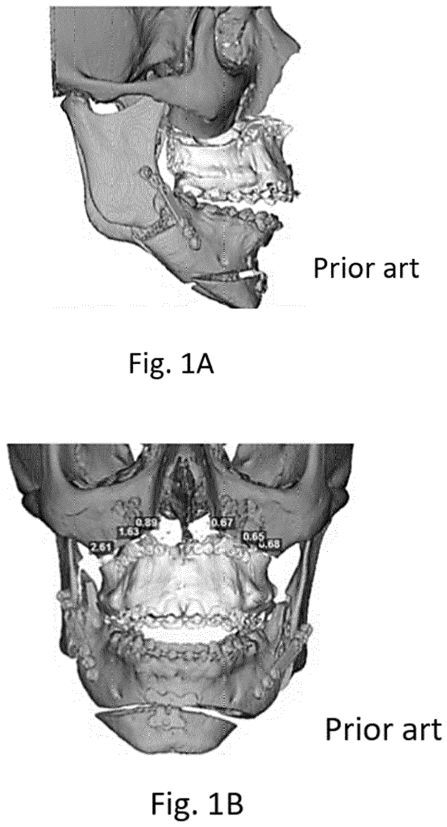

Moreover, although we can plan movements with accuracy of tenths of a millimetre, there is currently no objective system of intraoperative verification that the result obtained is the planned one in terms of the three-dimensional position of the facial skeleton, beyond manual measurements on the surgical field and the verification of occlusion by means of printed splints. Since there are difficulties in the surgical technique (e.g. condylar settlement, establishment of the maxillary vertical dimension, etc.), which can cause deviations from the planned positions, it is possible that the surgical result is not correct. Generally, the solution involves performing clinical and imaging controls in the postoperative period as shown in Fig. 1 and it is relatively frequently verified that the result is not exactly as planned, sometimes considering the need for reoperation. Fig. 1 shows an example of a postoperative image wherein the surgical results are not as planned. Obviously, this situation is not at all desirable and should be avoided, to the benefit of the patient and the health system.

There is therefore a need for a method to evaluate if a surgery is being performed according to the planned surgical procedure with the same precision as the planned surgical procedure was performed and with the least impact on the patient and on the resources.

### Summary of the invention

The present solution overcomes the above identified problem by providing a method for intraoperatively continuously monitor and correct the planned surgery.

According to a first aspect of the invention, there is provided a computer-implemented method for monitoring the spatial deviation with respect to a planned surgical procedure. The method is implemented through a system comprising at least a processor, the system characterised by the processor having access to a planned surgical procedure comprising one or more three-dimensional (3D) images corresponding to one or more predefined states of the planned surgical procedure in a body portion. The method comprises the following steps:
a) receiving one or more image of the body portion obtained by an intraoperative imaging device wherein the one or more image is associated to a certain predefined state of the planned surgical procedure;
b) superimposing the one or more received images of step a) with the corresponding section of the one or more 3D image of the planned surgical procedure associated to the same predefined state; and
c) determining the spatial deviation of the surgical procedure results with respect to the planned surgical procedure from the superimposition of the images of step b).

In a particular embodiment, the one or more received image of step a) is a computer tomography (CT) image and the body portion comprises at least one bone. Preferably, the planned surgical procedure comprises at least one bone relocation surgery, and the received image is associated to a certain predefined state of the planned surgical procedure after at least one bone relocation.

In another particular embodiment, the spatial deviation with respect to the planned surgical procedure is determined in a three-dimensional space.

In another particular embodiment, the method further comprises repeating the method steps until the spatial deviation is under a threshold, the threshold determining an acceptable deviation range from the planned surgical procedure.

In another particular embodiment, the system further comprises a imaging unit and the method further comprises displaying the superimposition of step b) .

In another particular embodiment, the system further comprises a imaging unit and the method further comprises displaying a colour-mapped representation of the spatial deviation determined in step c), preferably displaying it over the one or more 3D images of the planned surgical procedure and/or the one or more received images of step a).

In another particular embodiment, the superimposing of the images is performed based on a set of anatomical landmarks, wherein the equivalent landmarks of the one or more received images of step a) are aligned with the landmarks of the corresponding section of the one or more 3D image of the planned surgical procedure associated to the same predefined state. Preferably, the landmarks are provided over bones and comprise any of the list comprising: juxtapositions of tissues and maxima of curvatures.

In another particular embodiment, the one or more 3D images of the planned surgical procedure have been obtained through a computer tomography (CT) device.

In another particular embodiment, the one or more received images are 3D images.

In another particular embodiment, the surgery is an oral and/or cranio-maxillofacial and/or maxillofacial surgery.

In another particular embodiment, the surgery is a orthognathic surgery.

According to a second aspect of the invention, there is provided a computer program, comprising instructions configured to, when processed by a processor, perform the steps of a method for monitoring the spatial deviation with respect to a planned surgical procedure according to any of the methods of the first aspect of the invention.

According to a third aspect of the invention, there is provided a device comprising a processor configured to execute instructions to perform the steps of a method for monitoring the spatial deviation with respect to a planned surgical procedure according to any of the methods of the first aspect of the invention.

### Brief description of the drawings

To enable a better understanding of the present disclosure, and to show how the present disclosure may be carried out, reference will now be made, by way of example only, to the accompanying schematic drawings, wherein:
Figure 1 shows a postoperative three-dimensional representation of a human skull showing the surgery results according to the prior art.
Figure 2 shows a diagram of the method steps according to one or more embodiments of the present invention.
Figure 3 shows a diagram of the computer product according to one or more embodiments of the present invention.
Figure 4 shows a superimposition of the original position and the planned position of a maxilla viewed from (a) an horizontal view and (b) a perspective view of a cut-through sagittal view according to one or more embodiments.
Figure 5 shows a superimposition of the planned position and the position of the surgical procedure result of a maxilla viewed from (a) an horizontal view and (b) a perspective view of a cut-through sagittal view according to one or more embodiments.

### Description of the invention

### Definitions

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

It is noted that the term "about", as used herein, refers to +/- 30%, preferably +/- 20%, preferably +/- 15%, more preferably +/- 10%, of the indicated referred value.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of does not exclude materials or steps that do not materially affect the basic and novel characteristics of the claim.

The term "virtual surgery plan" refers to a preoperatory planification of a surgical procedure over a virtual representation of the body portion.

The term "superimposing" refers to spatially associating two images. In the context of the present invention is preferably understood as spatially associating the one or more received image to the one or more 3D image of the one or more 3D images of the planned surgical procedure associated to the same predefined state or to other received images associated to the same predefined state.

### Description

Each embodiment disclosed herein is contemplated as being applicable to each of the other disclosed embodiments. Thus, all combinations of the various elements described herein are within the scope of the invention. It should also be understood that, unless clearly indicated to the contrary, in any methods claimed herein that include more than one step or act, the order of the steps or acts of the method is not necessarily limited to the order in which the steps or acts of the method are recited

A first aspect of the invention refers to a computer implemented method for monitoring the spatial deviation with respect to a planned surgical procedure. Planned surgical procedures, usually involves a virtual surgery which is the design and planning of a surgery prior to the same based on anatomical images from the patient, wherein the surgeons can define the different steps and approaches during the surgery.

The method is implemented through a system comprising at least a processor. The processor may comprise one or more processing units, such as a microprocessor, GPU, CPU, multi-core processor or the like.

It therefore involves using preoperative images, usually three-dimensional (3D) images. While planned surgical procedures through images is common in complex surgical procedures, their use is limited to the preoperative stage of the procedure. Through the present method, the system is able to use the planned surgical procedure to monitor spatial deviations with respect to this planned surgical procedure which could be of use for surgeons performing such surgery.

The system is characterised by the processor having access to a planned surgical procedure comprising one or more three-dimensional (3D) images corresponding to one or more predefined states of the planned surgical procedure in a body portion. It is noted that these 3D images corresponding to one or more predefines states of the planned surgical procedure are obtained and/or generated prior to the surgical procedure. The one or more 3D images may comprise more than one image, for example when the images are obtained through different techniques, therefore allowing to identify different tissues, such as CT images for bones and MRI for soft tissues. It is therefore noted that the one or more 3D image can be obtained from different imaging techniques, such as CT, including cone beam CT (CBCT), MRI, PET, PET-CT scans, and other imaging techniques as the skilled person may derive. The one or more 3D images correspond to one or more predefined states of the planned surgical procedure, wherein each predefined state represents different steps of the planned surgical procedure. We note that surgeries and specially virtually planned surgeries, may comprise several milestones through the plan, each of the milestones usually defining the end of a certain step. Therefore, it may be defined that each of these milestones corresponds to a state of the planned surgical procedure. Therefore, the one or more 3D preoperative image may comprise both preoperative 3D images and virtually generated images, based on the planned surgical procedure. It is noted that when more than one preoperative 3D image is received, they may be images using the same or different techniques as some surgical steps may require analysing different tissues than other, for which different types of images may be required.

The one or more 3D images correspond to one or more predefined states of the planned surgical procedure may further comprises information on the planification. This planification may comprise information such as the identification of the different structures, the particular steps of the planning represented by such state, the points to interact with for the next state among other. The planification may comprise other different information according to the particular surgical plans and surgery performed as the skilled person may note.

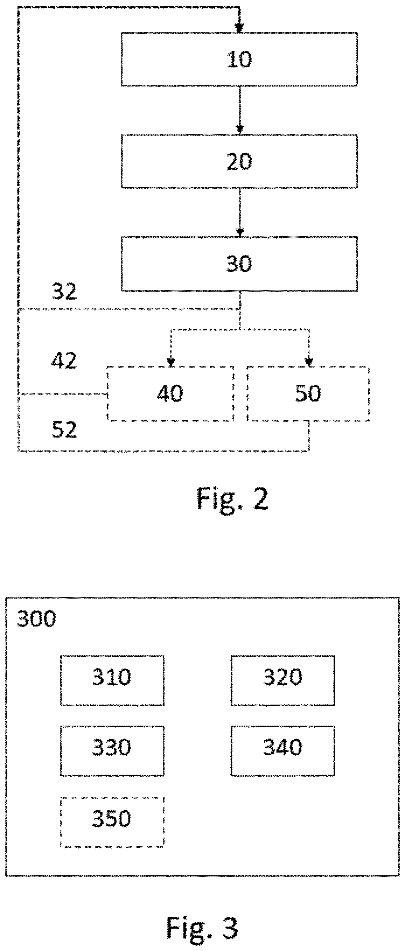

As shown in Fig. 2, the method comprises the following steps.

In a first step 10, the method involves receiving one or more image of the body portion obtained by an intraoperative imaging device wherein the one or more image is associated to a certain predefined state of the planned surgical procedure. The one or more received image of the body portion is provided by a intraoperative imaging device. Preferably, the received image has been taken through the surgical procedure after part of it has been performed, preferably after a certain step of interest of the planned surgery is completed. This allows the surgeons to determine during the surgery how to react to the deviations with respect to the planned surgical procedure. It is noted that the received image may be two-dimensional (2D) or three-dimensional (3D), and may be achieved, as the one or more 3D images of the planned surgical procedure through different means. Therefore, the one or more image obtained by an intraoperative imaging device can be obtained from different imaging devices, such as CT, MRI, PET, PET-CT scans, and other imaging devices as the skilled person may derive. For this purpose, specific scans for surgical environments, may be preferably used, which reduces the time taken to obtain the one or more received image, while simplifying the process as there is no need to leave the surgical room.

Secondly, as shown in step 20, the method comprises the step of superimposing the one or more received images of step 10 with the corresponding section of the 3D image of the planned surgical procedure associated to the same predefined state.

The superimposition implies spatially associating the one or more received image to the one or more 3D image of the planned surgical procedure associated to the same predefined state. To do so, several techniques may be used. For example, both the one or more received images and the one or more 3D images of the planned surgical procedure associated to the same predefined state may be obtained using a imaging device comprising a gyroscope or an inertial measurement unit (IMU) used in combination with a reference point. Therefore, each image can comprise information on the orientation and direction of the images. Alternatively, image recognition algorithms may be employed to recognise patterns on the images and create the spatial association between the one or more received images and the one or more 3D images of the planned surgical procedure associated to the same predefined state.

It is noted that the corresponding section for a received 2D image is not computed in the same way as for a received3D image. The equivalent section for a intraoperative 2D image is a cut plane through the one oi more 3D images. In order to determine this cut plane can be achieved as above determined, based on the direction and orientation of the imaging device with respect to the body portion. However, the equivalent section for a intraoperative 3D image is a portion or the whole 3D intraoperative image. However, the one or more received 3D image may need to be reoriented in order to be able to superimpose it over the one or more 3D image of the planned surgical procedure associated to the same predefined state. Moreover, the superimposing of a received 3D image allows to define multiple 2D cut planes and superimpose them with the 3D image of the planned surgical procedure associated to the same predefined state along such cuts, wherein the cuts are planes of interest. This allows to analyse different portions independently according to the needs of the surgeon. For example, the surgeon might require comparing a sagittal view of both images. The number of cut planes that can be defined is not limited, and can comprise many other relevant cuts according to the surgery, as the skilled person may note.

In both received 2D and 3D images, it might be required, in addition to rotation and orientation, to transform the images. For example, when the images are taken with different devices, it is common that the one or more received image and the one or more 3D image of the planned surgical procedure associated to the same predefined state have different resolutions, noise or log-compression values. Thus, prior to superimposing the images, it might be required to normalise both images, such that a proper superimposition can be achieved

Also, in both 2D and 3D received images, the one or more received image may comprise a extension over the body portion different to the extension of the one or more 3D image of the planned surgical procedure associated to the same predefined state. As long as they comprise part of the body portion, the superimposing can be achieved by superimposing such part of the body portion by the equivalent part of the body portion in the 3D image of the planned surgical procedure associated to the same predefined state.

Once the one or more received image and the one pr more 3D image of the planned surgical procedure associated to the same predefined state are associated, they can be superimposed, by representing each superimposed pair of images in different ways. For example, the one or more 3D image of the planned surgical procedure associated to the same predefined state can be represented as a solid image, and the corresponding section of the one or more received image can be shown as a translucent image over the same. The opposite can be also performed by representing the one or more received image as a solid and the corresponding section of the one or more 3D image of the planned surgical procedure associated to the same predefined state as a translucent image over the same. Alternatively or additionally, colours may be employed to identify each of the images. In another exemplary embodiment, the superimposition can be shown as the intersection of both images of the planned surgical procedure and received images, or as the symmetric difference of both planned surgical procedure and received images. These and other alternative superimposition ways as the skilled person may note are comprised within the present invention.

When the one or more 3D images of the planned surgical procedure associated to the same predefined state or the one or more received images comprise more than one image, it is noted that the superimposition may be performed between one or more of the different combination between the 3D images of the planned surgical procedure associated to the same predefined state and the images.

Finally, in a third step 30, the method comprises determining the spatial deviation of the surgical procedure results with respect to the planned surgical procedure from the superimposition of the images of step 20. The superimposition of step 20 enables the determination of the spatial deviation between the one or more 3D images of the planned surgical procedure and the one or more received image both associated to the same predefined state of the planned surgical procedure. The spatial deviation may be determined by characterising the superimposition based on different metrics such as distances, angles, or overlapping percentages.

Advantageously, this method provides a simple solution, wherein the planned surgical procedure can be used continuously for monitoring the spatial deviation which when the deviation is with respect to an image of a intraoperatory imaging device, if used during the surgery it allows to correct the surgery with the same precision as the planned surgical procedure. Also, it has the least impact on the patient, as there is no need for additional visits to the hospital, and the resource consumption is also diminished in terms of time and costs.

In some embodiments, the computer implemented method further comprises a fourth step of providing a treatment to a patient according to the spatial deviation of the surgical procedure results determined in the third step 30. For example, the treatment may be provided to the patient in which the planned surgery has been or is being performed, and the spatial deviation of the surgical procedure results determined in the third step 30 are used to provide such treatment, preferably to correct the surgical procedure results during the same procedure thus avoiding the need for further procedures. Advantageously, this provides a significant improvement in the patient experience, efficacy and resource management of the procedure compared to the need of a subsequent procedure when a bad result is identified postoperatively.

In a preferred embodiment the one or more received image is a CT image and the body portion comprises a bone. Advantageously, this allows to receive one or more images of the bone within the body portion to monitor its spatial deviation. The received CT image may be obtained through a regular CT scan, or more preferably through a surgical CT scan, configured to provide images in a short period of time in the reduced space of the surgical room while the surgery is still ongoing. Advantageously, the bone location within the body portion during the surgery after at least part of the planned surgery can be identified.

In a more preferred embodiment of the previous embodiment, the planned surgical procedure comprises at least one bone relocation surgery, and the received image is associated to a certain predefined state of the planned surgical procedure after at least one bone relocation. Advantageously, as the one or more received CT image shows the bone relocation, it helps determining the spatial deviation between the bone in the certain predefined state of the planned surgical procedure after such bone relocation and the one or more 3D image of the planned surgical procedure associated to the same predefine state, so that if the spatial definition is monitored during the surgery, the bone relocation can be assessed and corrected if necessary.

In another preferred embodiment, the spatial deviation with respect to the planned surgical procedure is determined in a three-dimensional space. It is noted that while this may be easily computed when a 3D image is received, it requires receiving at least two 2D images in order to be able to compute the deviation of the surgical procedure in a three-dimensional space. Advantageously, as the deviation can be identified in all axes of the space, if the deviation is determined during the surgery, the surgical procedure can be modified accordingly to this 3D deviation, ensuring the correction provided considers all the dimensions. If only one-dimensional or two-dimensional deviations of the surgical procedure were provided, it could not provide enough information to determine how to modify the virtual surgery, or it could lead to a bad modification, further leading to subsequent misalignments in other dimensions. Therefore, this is crucial for complex body portions, wherein multiple elements such as bones or muscles interact in a 3D space, for example in a joint. In these body portions, the whole picture has to be considered to avoid creating further deviations by solving the determined spatial deviation.

As shown with respect to Fig. 2, in another preferred embodiment of the first aspect of the invention, the method further comprises repeating the steps until the spatial deviation is under a threshold, the threshold determining an acceptable deviation range from the planned surgical procedure, as seen in references 32,42, 52. It is noted the method is repeated up to the last step according to such embodiment, so that if in a certain embodiment one or more subsequent steps 40, 50 of the method of continuous virtual surgery planning to correct the deviations from the plan during the same are also performed, those steps shall be also performed, as represented with reference 42.

Therefore, if the images are received and the deviation determined during a given part of the surgery, the step results can be analysed by computing the spatial deviation until an acceptable deviation range from the planned surgical procedure is achieved. Only then, the surgery may continue. This may be extremely useful in surgery plans wherein each step significantly conditions the next steps, as if the surgery results deviation are only analysed once, the corrected deviation may still be too high, therefore compromising the next steps.

The deviation threshold may be established in different ways. For example when the deviation is defined by more than one value, such as when the deviation is determined in a three-dimensional space, the threshold may be defined as the average or mean value of the different values defining the deviation. Alternatively or additionally, the values defining the deviation may be weighted differently according to different factors, such as the dimension or the relevance for a certain step. The deviation threshold values are defined in the same units as the deviation, including distances, angles, and/or overlapping percentages between both images.

In some embodiments, it may be determined that when a certain deviation is deemed as a minimum, i.e. that there is no correction that could be made leading to a smaller deviation, the deviation threshold is reached. Advantageously, this prevents an infinite look in those cases wherein the threshold cannot be reached.

In another preferred embodiment, the system further comprises a imaging unit and the method further comprises the step 40 displaying the superimposition of step 20. This involves the display of any of the superimpositions of step 20 or more than one. The display wherein the superimposition is displayed may be a screen an holographic medium or a projection of the image over an element, such as over the body portion of the patient. The display may be performed by any of the means such as displays, projectors, and other imaging devices such as virtual reality (VR) or augmented reality (AR) devices. The alternative displaying means the skilled person may note that may be used to display the superimposition are also comprised within the present embodiment of the invention.

It is noted, that as shown in Fig.2, when in this embodiment the method comprises repeating the steps until the spatial deviation is under a threshold as described with reference to the previous preferred embodiment, the method is repeated up to step 40, and only then is repeated, as shown with reference 42.

In another preferred embodiment, as shown in Fig. 2, the system further comprises a imaging unit and the method further comprises the step 50 of displaying a colour-mapped representation of the deviations. Advantageously, given a certain colour scale, a coloured-map provides an intuitive way of identifying the deviations and determining the type of deviation, such as positive or negative deviations, or bigger and smaller deviations. More preferably, the coloured-mapped representation is displayed over the one or more 3D image of the planned surgical and/or the colour-mapped representation is displayed over the one or more received image of step 10. Advantageously, if this is performed during the surgery of the received image, this further helps the surgeon to locate the deviation over the planned surgery and/or over the current results of the surgery, respectively.

It is noted, that as shown in Fig.2, when in this embodiment the method comprises repeating the steps until the spatial deviation is under a threshold as described with reference to the previous preferred embodiment, the method is repeated up to step 50, and only then is repeated, as shown with reference 52.

In another preferred embodiment, the superimposing of the images is performed based on a set of anatomical landmarks. In this embodiment, the equivalent landmarks of the one or more received images of step 10 are aligned with the landmarks of the corresponding section of the one or more 3D images of the planned surgical procedure associated to the same predefined state. The landmarks comprise any anatomical marks that can be defined over both the one or more 3D image of the planned surgical procedure associated to the same predefined state and the one or more image received in step 10. In the use of the landmarks to make the superimposition, the corresponding landmarks of each image are aligned. It may be understood that two landmarks are aligned when the images are located such that the landmarks are located in the same position or such that that the projection of a landmarks from an image over the other image along a certain direction is the same. When perfect alignment is not possible, some landmarks may be prioritized over other. For example, it may be considered that one dimension is more relevant than other, to preserve symmetry, and thus the alignment of the landmarks defining such dimension would be prioritized.

In another preferred embodiment, the superimposing of the images is performed based on a set of non-anatomical landmarks provided on the surgical guides or in the bones. These landmarks may comprise radiopaque elements such as metallic elements, For example, the set of landmarks may comprise a set of metallic balls comprised within the surgical guides. According to another example the non-anatomical landmarks may comprise a set of bolts comprised within the bones. In the use of the landmarks to make the superimposition, the corresponding landmarks of each image are aligned. It may be understood that two landmarks are aligned when the images are located such that the landmarks are located in the same position or such that that the projection of a landmarks from an image over the other image along a certain direction is the same. When perfect alignment is not possible, some landmarks may be prioritized over other. For example, it may be considered that one dimension is more relevant than other, to preserve symmetry, and thus the alignment of the landmarks defining such dimension would be prioritized.

It is noted that in some embodiments, the landmarks may be defined over reference elements that are considered to not have changed throughout the part of the surgery already performed, such that the landmarks serve as real coordinate systems to align both images. It is also noted that when the one or more received image is a 2D image, the landmarks of the received image are landmarks on the corresponding cut plane over the 3D image of the planned surgical procedure associated to the same predefined state.

In a more preferred embodiment, the landmarks are provided over bones of the images and comprise any landmark according to the Bookstein classification, preferably juxtapositions of tissues and/or maxima of curvatures. Advantageously, this allows to have a consistent frame of landmarks to track the displacement of the bones, which can serve to track the deviations of both the hard tissue as bones and soft tissue that is indirectly related to such bones, such as tendons and muscles attached to such bones.

In another preferred embodiment of the first aspect of the invention, the one or more 3D images of the planned surgical procedure have been obtained through a preoperative computer tomography (CT) scanner.

Advantageously, this allows to have a comprehensive 3D model of the body portion. As CT imaging provides a high-resolution 3D image of the bones of the subject, having the preoperative 3D image obtained through a CT scan provides highly cost-effective base model to perform the planned surgical procedure. To do so, the subject requires to take the one or more CT image before undergoing surgery. However, it is noted that a preoperative CT or CBCT scanner is usually already comprised as part of the protocol in order to plan a surgery in developed countries. This means that in the cases wherein a planned surgical procedure is required, this embodiment dos not involve more resource expenses nor bothering the patient.

In another preferred embodiment, the one or more image received in step 10 is a 3D image. Advantageously, this provides more information on the body portion, so that , when the results of this 3D image is compared with the preoperative 3D image, the spatial deviation can be determined in more points with the associated benefits of having more information. If the image is received during the surgery, it can provide more information on how to correct or adapt the surgical procedure. There are many ways the skilled person may note an 3D image obtained by an intraoperative imaging device may be taken, for example using an intraoperative CT scanner. When more than one intraoperative 3D image is received, this may be received from different imaging devices such as CT, RM, PET-TC scanners.

In another preferred embodiment, the surgery is an oral and/or cranio-maxillofacial and/or maxillofacial surgery. Oral, cranio-maxillofacial and maxillofacial surgeries are specifically benefitted from monitoring the deviation from the planned surgical procedure as described herein, as the region is both complex, involving multiple bones, and muscles with a complex jaw articulation, and it has a great aesthetic impact. Therefore precision in the surgical implementation of the plan, defined by the spatial deviation from it is highly valuable for both a correct functioning of the jaw and self-esteem of the patient.

In a more preferred embodiment of the previous embodiment, the surgery is an orthognathic surgery. As orthognathic surgery corrects irregularities of the jaw bones and realigns the jaws and teeth to improve the way they work, monitoring the deviation from the planned surgical procedure as described herein allows to ensure that the results achieve the expected results in terms of jaw functionality and/or appearance. The functionality of the jaw is extensive as it does not only affect chewing but can also affect speech, breath and can have indirect effects on sleep apnea and headaches among others. A correct orthognathic surgery result is therefore essential to ensure that all these functions are sustained.

A second aspect of the invention, refers to a computer program 300, as shown in Fig. 3. The computer program 300 comprises instructions configured to when processed by a processor, perform the steps of a method for monitoring the spatial deviation with respect to a planned surgical procedure according to any of the embodiments of the first aspect of the invention. The computer program 300 can be implemented in software, hardware, or a combination thereof and in some embodiments can be saved in a memory. The processor may comprise one or more processing units, such as a microprocessor, GPU, CPU, multi-core processor or the like. The memory may comprise one or more volatile or non-volatile memory devices, such as DRAM, SRAM, flash memory, read-only memory, ferroelectric RAM, hard disk drives, floppy disks, magnetic tapes, optical disks or the like. The computer program 300 may be configured to perform any of the steps of the method described in relation to Fig. 2.

The computer program 300 comprises an image receiving module 310 that is configured to receive the one or more 3D images corresponding to one or more predefined states of the planned surgical procedure and one or more images obtained by an intraoperative imaging device. The image receiving module is configured to connect to different image inputs from one or more processors, the processors configured to send the image to the image receiving module. The processor may be comprised within the same device as the computer program 300 and configured to provide the image to the image receiving module 310, for example from an external memory unit, or may be comprised within an external or cloud-based device. The processor may be comprised within the imaging device such as a CT scanner. The image receiving module may receive the one or more 3D images corresponding to one or more predefined states of the planned surgical procedure from a memory unit.

The computer program 300 also comprises an image processing module 320 that is configured to process the received images. The image processing module is 320 configured to process the images in order to make them suitable for the planned surgery procedure. For example, the imaging processing module 320 may be configured to identify tissues and/or scale, resample, cut, reorient, log-decompress and/or warp the different images received by the image receiving module 310 in order to make them compatible, Two images may not be compatible if they do not have the same resolution or if the log-compression is not the same. In order to perform an appropriate superimposition, the image processing module may be required to make these alterations to the images from the image received module 310. The image processing module may be further configured to analyse the metadata form the images in order to process them appropriately. In the embodiments wherein anatomical landmarks are used to superimpose the one or more image obtained by an intraoperatory imaging device associated to a certain predefined state with the one or more 3D image of the planned surgical procedure associated to the same predefined state, the image processing module 320 is further configured to identify the anatomical landmarks and note them.

The computer program 300 also comprises an superimposition module 330 that is configured to align the one or more 3D image of the planned surgical procedure with the one or more image obtained by an intraoperatory imaging device. The superimposition module 330 is configured to determine the equivalent section of both images. Therefore, when the one or more image obtained by an intraoperatory imaging device is a 2D image, the superimposition module 330 is configured to determine the cut plane on the one or more 3D image of the planned surgical procedure which is the equivalent section to such one or more 2D image obtained by an intraoperatory imaging device. In the embodiments wherein anatomical landmarks are used to superimpose the one or more 3D image of the planned surgical procedure with the one or more image obtained by an intraoperatory imaging device, the superimposition module 330 is configured to receive from the image processing module 320 the anatomical landmarks and use them to align the one or more 3D image of the planned surgical procedure with the one or more image obtained by an intraoperatory imaging device.

Optionally, the superimposition module 330 is further configured to receive information from a surgical navigation program to render the superposition of the one or more 3D image of the planned surgical procedure with the one or more image obtained by an intraoperatory imaging device or intraoperatively by any means. Advantageously, this allows the superimposition module 330 to show the superimposition in different views as the superimposition is navigated through the surgical navigation program.

The computer program 300 also comprises a deviation computation module 340, configured to calculate the deviation from the planned surgical procedure. The deviation computation module 340 is configured to request to the superimposition module 330 the superimposition of the one or more 3D image of the planned surgical procedure with the at one or more image obtained by an intraoperatory imaging device. From the superimposition the deviation computation module 340 is configured to compute the spatial deviation by characterising the superimposition based on different metrics such as distances, angles, or overlapping percentages.

In the embodiments wherein the deviation of the surgical procedure is determined in a three-dimensional space, the deviation computation module 340 is also configured to compute the deviation in the three dimensions.

In the embodiments wherein the method further comprises repeating the method steps until the spatial deviation is under a threshold, the deviation computation module 340 is also configured to define the threshold as the average or mean value of the different values defining the deviation. Alternatively or additionally, the deviation computation module 340 may weight the values defining the deviation according to different factors, such as the dimension or the relevance for a certain step. The deviation threshold values are defined in the same units as the deviation, including distances, angles, and/or overlapping percentages between both images.

When the processor is configured to display the superimposition or a colour-mapped representation of the spatial deviation, the computer program 300 also comprises an displaying module 350 that is configured to display the superimposition or a colour-mapped representation of the spatial deviation. The displaying module 350 is configured to send the superimposition representation or a colour-mapped representation of the spatial deviation to a display unit. The displaying module 350 may be configured to send the superimposition or a colour-mapped representation of the spatial deviation to different display units, such as to a screen, a projector unit, an holographic system, a virtual reality system or an augmented reality system. The displaying module 350 may be configured to adapt the superimposition or a colour-mapped representation of the spatial deviation according to the type of display by instructing the image processing module 320, for example to perform a downscaling or the decomposition of an image, for example to represent different elements at different depths in an holographic medium.

A third aspect of the invention refers to a device 400 comprising a processor 410 configured to execute instructions to perform the steps of a method for monitoring the spatial deviation with respect to a planned surgical procedure according to any of the embodiments of the first aspect of the invention. The instructions may be saved in a memory 420 comprised within the product or in an external memory. The memory may comprise one or more volatile or non-volatile memory devices, such as DRAM, SRAM, flash memory, read-only memory, ferroelectric RAM, hard disk drives, floppy disks, magnetic tapes, optical disks or the like. Likewise, the processor may comprise one or more processing units, such as a microprocessor, GPU, CPU, multi-core processor or the like.

All of the above are fully within the scope of the present disclosure and are considered to form the basis for alternative embodiments in which one or more combinations of the above-described features are applied, without limitation to the specific combination disclosed above.

In light of this, there will be many alternatives which implement the teaching of the present disclosure. It is expected that one skilled in the art will be able to modify and adapt the above disclosure to suit its own circumstances and requirements within the scope of the present disclosure, while retaining some or all technical effects of the same, either disclosed or derivable from the above, in light of his common general knowledge in this art. All such equivalents, modifications or adaptations fall within the scope of the present disclosure.

### EXAMPLE

### Material and methods

A maxillofacial surgical procedure was planned. TAC images were obtained and a 3D model was built through image 3D mapping techniques.

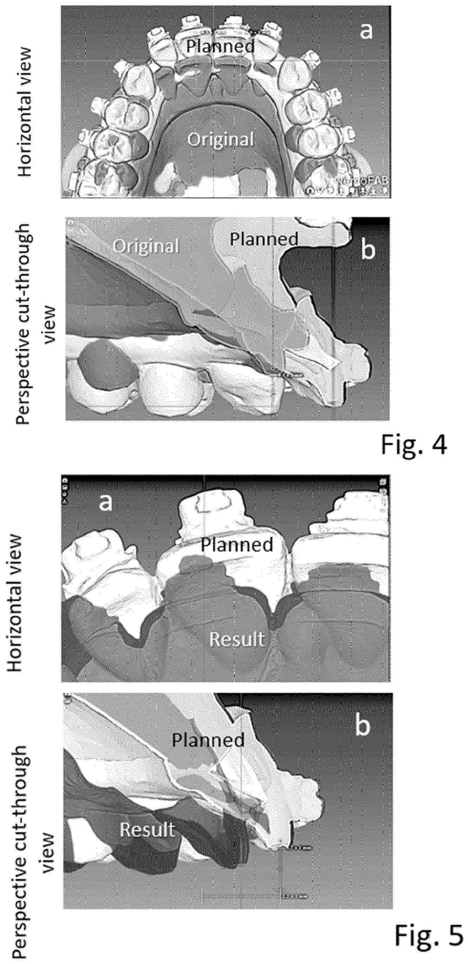

As shown in Fig. 4A and 4B, the surgical procedure was planned, and a virtual position of the bone tissue of the patient was generated. By comparing the 3D model of the patient's maxilla and the planned position of the maxilla after the procedure, the following movements were planned:

| **Maxilla** | **A-P** | **Vertical** | **Transverse** |
|---|---|---|---|
| Mx1R tip | 5.9 | 3.1 | 0 |
| Mx1L tip | 5.9 | 2.9 | 0 |
| Right Molar 6 MB cusp tip | 5.9 | 0.8 | 0 |
| Left Molar 6 MB cusp tip | 6 | 2.3 | 0 |

### Results

After the procedure, an intraoperative TAC image was obtained. As shown in Figs. 5A and 5B, the planned position of the maxilla after the procedure, and the results from the procedure were compared and the following discrepancies were obtained:

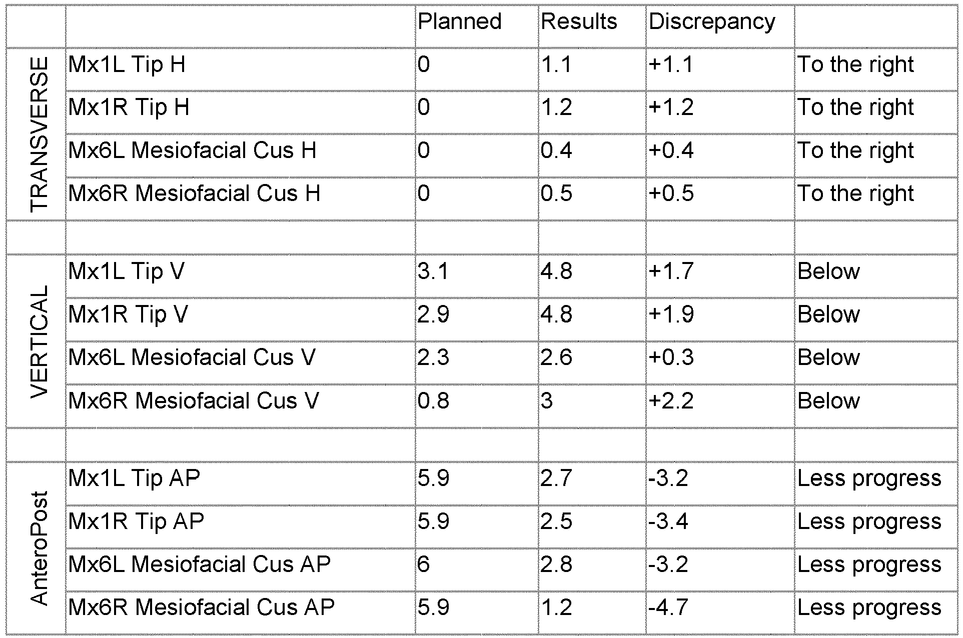

It can be noted that there have been unwanted transverse movements and that the surgical position of the maxilla registers a greater decrease and less progress than desired. The numerical values of the reference points analysed are given in the table: in the first column, the planned, in the second the obtained and in the third the difference.

## Claims

1. A computer-implemented method for monitoring the spatial deviation with respect to a planned surgical procedure, wherein the method is implemented through a system comprising at least a processor, the system **characterised by** the processor having access to a planned surgical procedure comprising one or more three-dimensional (3D) images corresponding to one or more predefined states of the planned surgical procedure in a body portion, wherein the method comprises the following steps:
a) receiving one or more image of the body portion obtained by an intraoperative imaging device wherein the one or more image is associated to a certain predefined state of the planned surgical procedure;
b) superimposing the one or more received images of step a) with the corresponding section of the one or more 3D image of the planned surgical procedure associated to the same predefined state; and
c) determining the spatial deviation of the surgical procedure results with respect to the planned surgical procedure from the superimposition of the images of step b).

2. The method according to claim 1, wherein the one or more received image of step a) is a computer tomography (CT) image and the body portion comprises at least one bone.

3. The method according to claim 2, wherein the planned surgical procedure comprises at least one bone relocation surgery, and wherein the received image is associated to a certain predefined state of the planned surgical procedure after at least one bone relocation.

4. The method according to any of the claims 1 to 3, wherein the spatial deviation with respect to the planned surgical procedure is determined in a three-dimensional space.

5. The method according to any of the claims 1 to 4, wherein the method further comprises repeating the method steps until the spatial deviation is under a threshold, the threshold determining an acceptable deviation range from the planned surgical procedure.

6. The method according to any of the claims 1 to 5, wherein the system further comprises a imaging unit and the method further comprises displaying the superimposition of step b) .

7. The method according to any of the claims 1 to 6, wherein the system further comprises a imaging unit and wherein the method further comprises displaying a colour-mapped representation of the spatial deviation determined in step c), preferably displaying it over the one or more 3D images of the planned surgical procedure and/or the one or more received images of step a).

8. The method according to any of the claims 1 to 7, wherein the superimposing of the images is performed based on a set of anatomical landmarks, wherein the equivalent landmarks of the one or more received images of step a) are aligned with the landmarks of the corresponding section of the one or more 3D image of the planned surgical procedure associated to the same predefined state.

9. The method according to claim 8, wherein the landmarks are provided over bones and comprise any of the list comprising: juxtapositions of tissues and maxima of curvatures.

10. The method according to any of the claims 1 to 9, wherein the one or more 3D images of the planned surgical procedure have been obtained through a computer tomography (CT) device.

11. The method according to any of the claims 1 to 10, wherein the one or more received images are 3D images.

12. The method according to any of the claims 1 to 11, wherein the surgery is an oral and/or cranio-maxillofacial and/or maxillofacial surgery.

13. The method according to claim 12, wherein the surgery is a orthognathic surgery.

14. A computer program, comprising instructions configured to, when processed by a processor, perform the steps of a method for monitoring the spatial deviation with respect to a planned surgical procedure according to any of the methods of claims 1 to 13.

15. A device comprising a processor configured to execute instructions to perform the steps of a method for monitoring the spatial deviation with respect to a planned surgical procedure according to any of the claims 1 to 13.
